# EUROPÄISCHE PATENTSCHRIFT

(11) **EP 1 567 079 B1**
(45) Veröffentlichungstag und Bekanntmachung des Hinweises auf die Patenterteilung: **16.08.2006**
(21) Anmeldenummer: 03767747.3
(22) Anmeldetag: 04.12.2003
(51) Int. Cl.: A61B 18/14

(54) **BIPOLARES MEDIZINISCHES INSTRUMENT SOWIE ELEKTROCHIRURGISCHES SYSTEM MIT EINEM SOLCHEN INSTRUMENT**
BIPOLAR MEDICAL INSTRUMENT AND ELECTROSURGICAL SYSTEM COMPRISING ONE SUCH INSTRUMENT
INSTRUMENT MEDICAL BIPOLAIRE ET SYSTEME ELECTROCHIRURGICAL COMPORTANT UN TEL INSTRUMENT

(30) Priorität: 06.12.2002 DE 10258730
(43) Veröffentlichungstag der Anmeldung: 31.08.2005
(73) Patentinhaber: Karl Storz GmbH & Co. KG, 78532 Tuttlingen (DE)
(72) Erfinder: BLOCHER, Martin, 78532 Tuttlingen (DE)
(74) Vertreter: Heuckeroth, Volker
(86) Internationale Anmeldenummer: PCT/EP2003/013736
(87) Internationale Veröffentlichungsnummer: WO 2004/052220

(56) Entgegenhaltungen:
- EP-A- 1 163 886
- WO-A-99/16371
- DE-A- 2 521 719
- US-A- 2 056 377
- US-A- 5 419 767

## Beschreibung

Die Erfindung betrifft ein bipolares medizinisches Instrument zum Schneiden von Gewebe unter der Wirkung von Hochfrequenzstrom, mit einem sich längserstreckenden Elektrodenträger, einer an dem Elektrodenträger distalseitig angeordneten Aktivelektrode und einer dieser benachbarten Neutralelektrode, wobei die Aktivelektrode als Drahtschlinge ausgebildet ist, und wobei die Neutralelektrode vollständig distalseitig der Aktivelektrode angeordnet ist.

Die Erfindung betrifft ferner ein elektrochirurgisches System, mit einem Hochfrequenzgenerator und einem Instrument der eingangs genannten Art.

Ein Instrument und ein System der eingangs genannten Art sind aus der DE-OS 25 21 719 bekannt.

Ein Instrument bzw. ein System der eingangs genannten Art wird in der offenen, vorzugsweise aber in der minimal-invasiven Chirurgie zum Schneiden von Gewebe im menschlichen oder tierischen Körper verwendet.

Das eingangs genannte Instrument kann im Sinne der vorliegenden Erfindung bei Verwendung in der minimal-invasiven Chirurgie zusammen mit einem Endoskop zu einem sogenannten Resektoskop kombiniert werden oder selbst ein solches Resektoskop darstellen.

Die Elektro- oder Hochfrequenzchirurgie wird therapeutisch in verschiedenen medizinischen Fachdisziplinen wie bspw. der Urologie, Gynäkologie, Neurochirurgie, Abdominalchirurgie usw. eingesetzt. Speziell in der Urologie wird mittels eines eingangs genannten Instruments im Rahmen der minimal-invasiven Chirurgie endoskopisch Prostatagewebe abgetragen.

Bei elektrochirurgischen Verfahren der Resektoskopie unterscheidet man zwischen der monopolaren und der bipolaren Applikation des Hochfrequenzstromes.

Bei der monopolaren Applikation wird nur die Aktivelektrode in das Behandlungsgebiet eingeführt, während die Neutralelektrode außen am Patienten angeordnet wird. Folglich führt der Stromfluss zwischen der Aktivelektrode und der Neutralelektrode durch den Körper des Patienten hindurch, wobei der Nachteil besteht, dass der Strompfad durch den Patienten nicht sicher kontrolliert werden kann mit der Folge möglicher Verletzungen von Organen. Darüber hinaus kann die am Patientenkörper angebrachte Neutralelektrode zu Verbrennungen der Haut des Patienten führen.

Bei der bipolaren Technik, von der die vorliegende Erfindung ausgeht, werden sowohl Aktivelektrode als auch Neutralelektrode in das Behandlungsgebiet eingeführt. Der Stromfluss kann dadurch in kontrollierbarer Weise auf den Bereich zwischen der Aktivelektrode und der Neutralelektrode begrenzt werden. Entsprechend sind medizinische Instrumente der eingangs genannten Art geschaffen worden, bei denen die Aktivelektrode und die Neutralelektrode an einem Elektrodenträger angeordnet sind, so dass die Aktivelektrode und die Neutralelektrode einander benachbart in das Behandlungsgebiet eingebracht werden können.

Bei bipolaren medizinischen Instrumenten der eingangs genannten Art ist die Aktivelektrode üblicherweise mit einer kleinen wirksamen Oberfläche ausgebildet, so dass sich an der Aktivelektrode eine hohe Stromdichte einstellt, während die Neutralelektrode üblicherweise relativ großflächig ausgebildet ist, so dass sich an der Neutralelektrode nur mittlere oder geringe Stromdichten einstellen. Mit der Aktivelektrode wird entsprechend geschnitten, während die Neutralelektrode möglichst keine Wirkung auf das Gewebe ausüben soll, sondern lediglich der Begrenzung des Strompfades auf den Bereich zwischen der Aktivelektrode und der Neutralelektrode dienen soll.

Bei dem aus der oben genannten DE-OS 25 21 719 bekannten Instrument ist die Neutralelektrode als Bandschlinge ausgebildet, während die Aktivelektrode als Drahtschlinge ausgebildet ist. Die Neutralelektrode befindet sich dabei vollständig distalseitig der Aktivelektrode und verläuft zu dieser parallel. Bei diesem bekannten Instrument kann es vorkommen, dass beim Ansetzen des Instruments an das zu behandelnde Gewebe zuerst die Neutralelektrode das Gewebe berührt, bevor die Aktivelektrode das Gewebe berührt, mit der Folge, dass bei Beaufschlagung der Elektroden mit Hochfrequenzstrom das mit der Neutralelektrode in Berührung stehende Gewebe möglicherweise kauterisiert oder zumindest beschädigt wird, obwohl dieses Gewebe nicht behandelt werden sollte. Aber auch beim gleichzeitigen Berühren der Aktivelektrode und Neutralelektrode kann mit der Neutralelektrode in Berührung stehendes Gewebe, das nicht der Wirkung des Hochfrequenzstromes unterzogen werden soll, beschädigt werden.

In einem weiteren Ausführungsbeispiel der DE-OS 25 21 719 ist die Neutralelektrode auf ihrer der Aktivelektrode abgewandten Seite von einem Kunststoff-Fortsatz, der fest mit dem Schaft des Resektoskops verbunden ist, überdeckt, so dass das vorstehend genannte Problem einer Berührung von unbeteiligtem Gewebe mit der Neutralelektrode vermieden wird. Hierbei ist jedoch von Nachteil, dass die Neutralelektrode relativ zum Schaft des Resektoskops unbeweglich ist. Bei Resektoskopen kann es jedoch sinnvoll sein, die Neutralelektrode zusammen mit der Aktivelektrode relativ zum Schaft des Resektoskops zu bewegen, um den Schneidvorgang auszuführen, während die Endoskopoptik beim Schneidvorgang möglichst nicht bewegt werden soll, um den Blickwinkel bzw. das Gesichtsfeld beim Schneiden nicht zu verändern, um eine möglichst genaue optische Kontrolle des Schneidvorgangs zu erhalten. Darüber hinaus ist bei diesem Ausführungsbeispiel die Neutralelektrode proximalseitig der Aktivelektrode angeordnet, oder erstreckt sich sowohl proximalseitig als auch distalseitig von der Aktivelektrode.

Das Dokument WO-A-99 16371 offenbart ebenfalls ein bipolares medizinisches Instrument der eingangs genannten Art. Ähnlich zu dem zuvor beschriebenen bekannten Instrument ist die Aktivelektrode zur Neutralelektrode in Richtung quer zur Längsachse so positioniert, dass diese nicht voneinander beabstandet sind.

Das Dokument US-A-5,419,767 offenbart einen Katheter, der nahe dem distalen Ende des Katheters ein Elektroden-Array aufweist. Die distalseitige Elektrode ist in Form einer langerstreckten Katheternadel ausgebildet, während das Elektroden-Array proximalseitig davon konzentrisch um die distale Elektrode umfänglich verteilt angeordnet ist.

Des weiteren ist aus der EP 1 163 886 A2 ein resektoskopisches Instrument bekannt, bei dem die Aktivelektrode und die Neutralelektrode auf ihren einander zugewandten Seiten durch einen Isolatorkörper elektrisch getrennt sind, so dass jede gerade Verbindungslinie zwischen den Elektroden durch den Isolatorkörper verläuft. Dadurch soll der direkte Stromfluss zwischen den beiden Elektroden erschwert bzw. vermindert werden, wodurch ein größerer Anteil der in das Instrument eingespeisten Stromleistung von der Aktivelektrode in das Körpergewebe übergehen und dort Schneidwirkung entfalten soll. Der Isolatorkörper ist bei diesem Instrument auf der der Aktivelektrode zugewandten Innenseite der Neutralelektrode angeordnet und kann sich entsprechend bei einer Positionsveränderung der Neutralelektrode mit dieser mitbewegen.

Bislang hat sich keines der bekannten Instrumente hinsichtlich seiner Schneidwirkung als zufriedenstellend erwiesen.

Der Erfindung liegt die Aufgabe zu Grunde, ein Instrument sowie ein System der eingangs genannten Art bereitzustellen, mit dem mittels Hochfrequenzstrom Gewebe zufriedenstellend geschnitten werden kann.

Erfindungsgemäß wird diese Aufgabe hinsichtlich des eingangs genannten Instruments dadurch gelöst, dass die Neutralelektrode und die Aktivelektrode ohne einen räumlichen Überlappungsbereich voneinander in Richtung quer zur Längsrichtung des Elektrodenträgers beabstandet sind.

Ein erfindungsgemäßes elektrochirurgisches System der eingangs genannten Art weist entsprechend ein medizinisches Instrument der vorstehend genannten Art auf.

In Versuchen hat sich herausgestellt, dass das erfindungsgemäße bipolare medizinische Instrument sehr gute Schneidergebnisse liefert. Insbesondere, wenn das Instrument in einem Behandlungsareal verwendet wird, in dem sich eine elektrisch leitende Flüssigkeit befindet, bspw. wenn während der Resektion mit einer elektrolytreichen, bspw. isotonischen, Spülflüssigkeit gespült wird, werden hervorragende Schneidergebnisse erzielt. Auf Grund der in Richtung quer zur Längsrichtung des Elektrodenträgers beabstandeten Anordnung der distalseitig von der Aktivelektrode positionierten Neutralelektrode, die sich in Zugrichtung der Aktivelektrode beim Schneiden von Gewebe demnach hinter und über der Aktivelektrode befindet, hat sich dabei überraschenderweise als sehr wirksam hinsichtlich des Schneidens von Gewebe herausgestellt.

Ein weiterer Vorteil des erfindungsgemäßen medizinischen Instruments kann darin gesehen werden, dass die distalseitig der Aktivelektrode und bezüglich der Aktivelektrode in Richtung quer zur Längsrichtung des Elektrodenträgers versetzte Anordnung der Neutralelektrode die Sicht auf die Aktivelektrode beim Arbeiten mit dem Instrument nicht verdeckt, insbesondere, wenn das Instrument zusammen mit einer Endoskopoptik verwendet wird. Darüber hinaus besteht ein Vorteil darin, dass beim Schneiden von Gewebe mit der Aktivelektrode, bei dem die Aktivelektrode und damit die Neutralelektrode nach proximal zurückgezogen wird, die Neutralelektrode mit dem Schaft des Instruments nicht in Berührung kommt, so dass für den Schaft keine zusätzlichen Isolationsmaßnahmen erforderlich sind, sondern der Schaft kann genau so wie bei einem monopolaren Instrument ausgebildet sein.

Beim Ansetzen der Aktivelektrode an das zu schneidende Gewebe kommt die Neutralelektrode auf Grund der Beabstandung in Richtung quer zur Längsrichtung des Elektrodenträgers nicht mit dem zu schneidenden Gewebe in Kontakt.

In einer bevorzugten Ausgestaltung des Instruments ist die Aktivelektrode als Drahtschlinge ausgebildet, und alternativ oder kumulativ ist die Neutralelektrode als Bandschlinge ausgebildet, vorzugsweise mit von der Aktivelektrode weg gerichteter Krümmung.

Mit dieser Ausgestaltung des erfindungsgemäßen Instruments haben sich in Testversuchen besonders gute Schneidwirkungen des erfindungsgemäßen Instruments erzielen lassen.

In einer weiteren bevorzugten Ausgestaltung ist am Elektrodenträger und/oder an der Neutralelektrode zumindest ein Element vorhanden, das eine elektrisch leitende Berührung einer der Aktivelektrode abgewandten Außenseite der Neutralelektrode durch Gewebe verhindert, und das sich bei einer Positionsveränderung der Neutralelektrode mit dieser mitbewegt.

Das zumindest eine Element wirkt in der Art eines Abstandshalters zwischen der Neutralelektrode und unbeteiligtem Gewebe, wodurch eine Beschädigung unbeteiligten Gewebes bei der Handhabung des Instruments im Behandlungsbereich vermieden wird, was insbesondere beim Einsatz des Instruments in engen Behandlungsbereichen von Vorteil ist, da der das Instrument bedienende Arzt dann nicht ständig Sorge dafür tragen muss, die Neutralelektrode von unbeteiligtem Gewebe abzuhalten.

In einer weiteren bevorzugten Ausgestaltung ist das zumindest eine Element selbst elektrisch isolierend.

Hierbei ist von Vorteil, dass eine elektrisch leitende Berührung der Neutralelektrode mit Gewebe ohne komplizierte konstruktive Maßnahmen sicher vermieden werden kann, und dass das Element unmittelbar an der Neutralelektrode befestigt werden kann.

In einer weiteren bevorzugten Ausgestaltung ist das zumindest eine Element aus einem keramischen Werkstoff oder aus einem Kunststoff.

Diese Maßnahme hat den Vorteil, dass die vorstehend genannten Materialien kostengünstig sind. Als Kunststoffe können bspw. unter der Marke Safecoat 720 vertriebene Materialien verwendet werden, oder bspw. Polytetrafluorethylen. Als keramischer Werkstoff kann bspw. Zirkonoxid verwendet werden.

In einer weiteren bevorzugten Ausgestaltung ist das zumindest eine Element durch eine Beschichtung der Neutralelektrode gebildet.

Hierbei ist von Vorteil, dass das Element, das eine Berührung der Neutralelektrode durch unbeteiligtes Gewebe verhindert, relativ dünn auf die Neutralelektrode aufgebracht werden kann. Als Beschichtungsmaterial eignet sich hierbei insbesondere Polyetheretherketon (PEEK), das den Vorteil hat, hochtemperaturfest und mechanisch beständig, insbesondere schlagzäh, zu sein. Damit ist die Neutralelektrode beim Autoklavieren und beim Gebrauch des Instruments gegen Beschädigungen geschützt.

In einer weiteren bevorzugten Ausgestaltung ist das zumindest eine Element unmittelbar auf der der Aktivelektrode abgewandten Außenseite der Neutralelektrode befestigt.

Hierbei ist von Vorteil, dass das zumindest eine als Abstandshalter wirkende Element die Querabmessung der Elektrodenanordnung nicht wesentlich vergrößert, so dass sich das Instrument insbesondere auch für die minimal-invasive Chirurgie, in der miniaturisierte Instrumente gefordert werden, besonders eignet.

In einer weiteren bevorzugten Ausgestaltung bedeckt das zumindest eine Element die Außenseite der Neutralelektrode vollflächig.

Hierbei ist von Vorteil, dass das zumindest eine Element mit einer sehr geringen Dicke auf der Außenseite der Neutralelektrode aufgebracht werden kann, so dass eine elektrisch leitende Berührung der Neutralelektrode mit Gewebe nicht durch eine räumliche Beabstandung vermieden werden muss.

In einer weiteren bevorzugten Ausgestaltung verhindert das zumindest eine Element eine elektrisch leitende Berührung der distalseitigen und/oder proximalseitigen Stirnseite der Neutralelektrode mit Gewebe.

Hierbei ist von Vorteil, dass auch beim Vor- und Zurückbewegen des Elektrodenträgers und damit der Aktivelektrode und der Neutralelektrode ein Kontakt der Neutralelektrode mit Gewebe sicher vermieden werden kann.

Dabei ist es wiederum bevorzugt, wenn das zumindest eine Element unmittelbar auf der der Aktivelektrode abgewandten Außenseite der Neutralelektrode befestigt ist und dabei die distalseitige und/oder proximalseitige Stirnseite der Neutralelektrode vollflächig bedeckt.

In einer weiteren bevorzugten Ausgestaltung des elektrochirurgischen Systems ist der Hochfrequenzgenerator in der Lage, eine Ausgangsleistung von zumindest 200 Watt abzugeben.

Gerade die Kombination des erfindungsgemäßen Instruments mit einem solchen Hochfrequenzgenerator hat sich in der Praxis als besonders wirksam erwiesen, was darauf beruht, dass auf Grund der Beabstandung der Neutralelektrode von der Aktivelektrode zunächst nur die Aktivelektrode mit dem zu schneidenden Gewebe in Berührung kommt, während die sich in Zugrichtung betrachtete hintere Neutralelektrode sich noch innerhalb der Spülflüssigkeit befindet und somit der Strom größtenteils nur durch die isotonische Kochsalzlösung fließt, nicht jedoch durch das Gewebe. Mit der höheren Leistung des Hochfrequenzstroms kann dieses Problem jedoch überwunden werden, wie sich in der Praxis gezeigt hat.

Besonders bevorzugt ist es, wenn der Hochfrequenzgenerator eine Leistungsregelung aufweist, derart, dass die von dem Hochfrequenzgenerator abgegebene Leistung vor und während des Eindringens der Aktivelektrode in Gewebe (Anschneiden) höher ist als beim Weiterschneiden, wenn die Aktivelektrode in das Gewebe eingedrungen ist.

Wie vorstehend erläutert wurde, kann das Anschneideverhalten des Instruments entscheidend verbessert werden, wenn die Schneidleistung auf Werte über 200 Watt erhöht wird. Würde man jedoch diese hohe Ausgangsleistung des Hochfrequenzgenerators während des ganzen Schneidvorganges beibehalten, würde dies zumindest in der Anfangsphase des Schnittes zu einer sichtbaren Carbonisierung des Gewebes führen, die unerwünscht ist. Dieser Nachteil kann dadurch vermieden werden, dass die Leistung des Hochfrequenzstroms entsprechend beim Weiterschneiden verringert wird.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Fig. 1: Ein elektrochirurgisches System mit einem bipolaren medizinischen Instrument und einem äußerst schematisch dargestellten Hochfrequenzgenerator, wobei das Instrument in Seitenansicht dargestellt ist;
- Fig. 2: eine Elektrodenanordnung des medizinischen Instruments in Fig. 1 in Seitenansicht und gegenüber Fig. 1 vergrößertem Maßstab;
- Fig. 3: die Elektrodenanordnung in Fig. 2 in Draufsicht;
- Fig. 4: einen distalen Bereich des Instruments in Fig. 1 in Seitenansicht und noch weiter vergrößertem Maßstab;
- Fig. 5: eine Vorderansicht auf das Instrument in Fig. 1 in stark vergrößertem Maßstab; und
- Figuren 6a) und b): das distale Ende des Instruments in Fig. 1 in vergrößertem Maßstab, wobei Fig. 6a) den Anschneidvorgang und Fig. 6b) den Weiterschneidvorgang veranschaulicht.

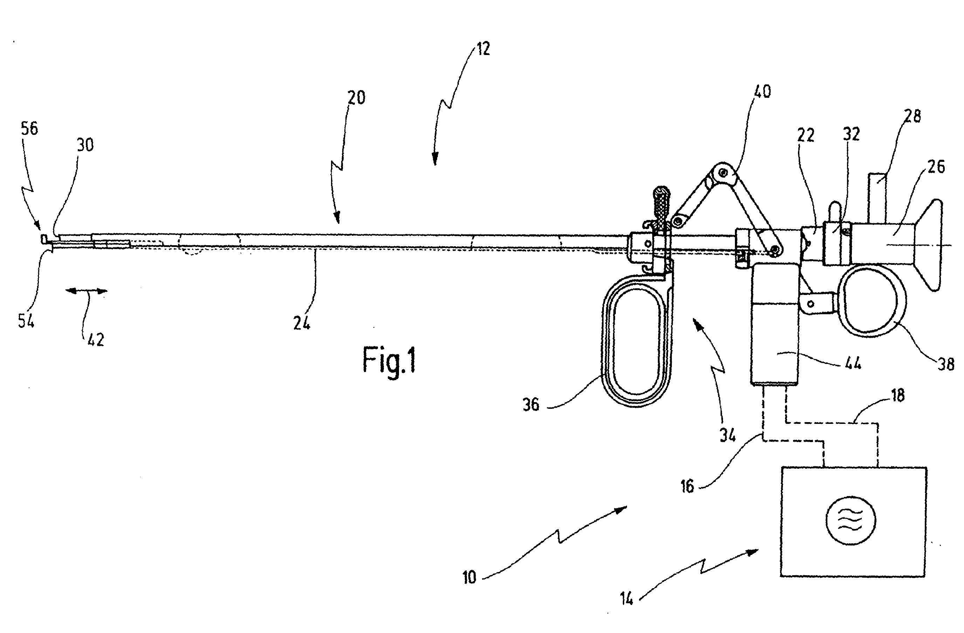

In Fig. 1 ist ein mit dem allgemeinen Bezugszeichen 10 versehenes elektrochirurgisches System dargestellt, das im Rahmen der minimal-invasiven Resektoskopie verwendet wird.

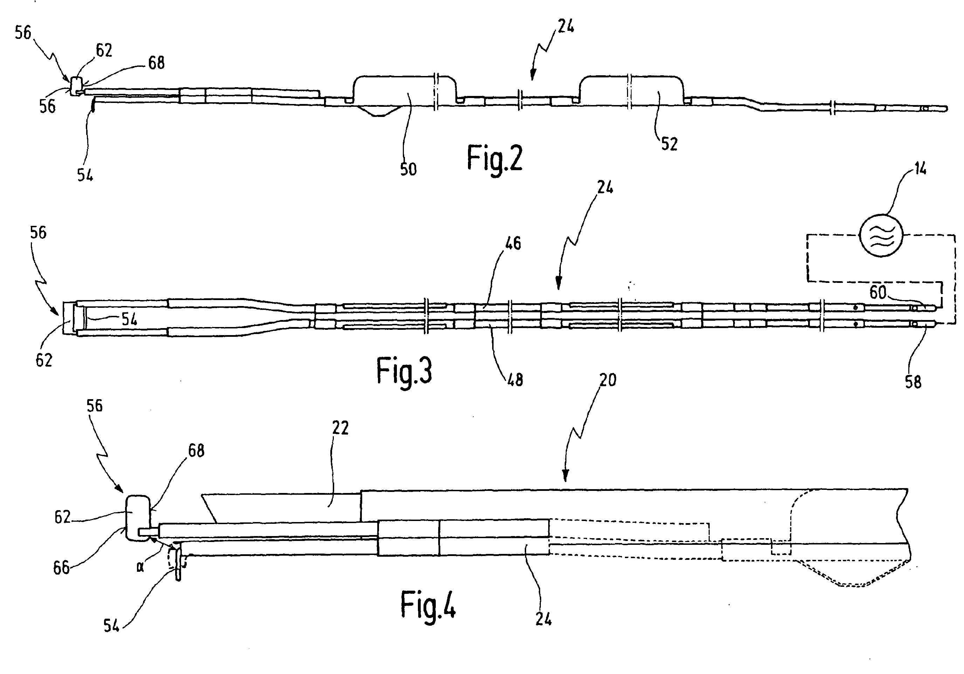

Das System 10 weist ein bipolares medizinisches Instrument 12 in Form eines Resektoskops auf, von dem in Figuren 2 bis 5 weitere Einzelheiten dargestellt sind.

Das System 10 umfasst ferner einen Hochfrequenzgenerator 14, der in Fig. 1 äußerst schematisch dargestellt ist. Der Hochfrequenzgenerator 14 erzeugt Hochfrequenzstrom, bzw. Hochfrequenzspannung, der bzw. die dem Instrument 12 über Leitungen 16 und 18, von denen eine mit dem aktiven Pol des Hochfrequenzgenerators 14 und die andere mit dem inaktiven Pol des Hochfrequenzgenerators 14 verbunden ist, zugeführt wird.

Nachfolgend wird zunächst das Instrument 12 näher beschrieben.

Das Instrument 12 dient zum Schneiden von Gewebe im menschlichen oder tierischen Körper unter der Wirkung von Hochfrequenzstrom.

Das Instrument 12 weist einen langerstreckten Schaft 20 auf, in dem eine Endoskopoptik 22 und ein Elektrodenträger 24 angeordnet sind.

Die Endoskopoptik 22 weist an ihrem proximalen Ende ein Okular 26 und einen Anschluss 28 zum Anschließen eines Lichtleitkabels zur Zuführung von Beleuchtungslicht in die Endoskopoptik 22 auf. Die Endoskopoptik 22 erstreckt sich distal bis zu einem lichtaustrittsseitigem Ende 30, das mit einer Schrägblickoptik versehen ist.

Die Endoskopoptik 22 ist über eine Kupplung 32 mit dem Schaft 20 des Instruments 12 verbunden und kann durch Lösen der Kupplung 32 vom Instrument 12 abgenommen werden.

Ferner weist das Instrument 12 im proximalen Bereich eine Handhabe 34 mit einem Fingergriffteil 36 und einem Daumengriffteil 38 auf, wobei das Daumengriffteil 38 über ein Scharniergelenk 40 relativ zu dem Fingergriffteil 36 beweglich ist. Das Fingergriffteil 36 und das Daumengriffteil 38 können relativ zueinander aufeinander zu- und voneinander wegbewegt werden. Durch entsprechendes Betätigen der Handhabe 34 kann der Elektrodenträger 24 relativ zum Schaft und relativ zur Endoskopoptik 22 in den Richtungen eines Doppelpfeiles in Längsrichtung des Schaftes 20 bewegt werden.

Schließlich ist im proximalen Bereich des Instruments 12 ein Steckeranschluss 44 angeordnet, über den das Instrument 12 mit dem Hochfrequenzgenerator 14 verbunden werden kann.

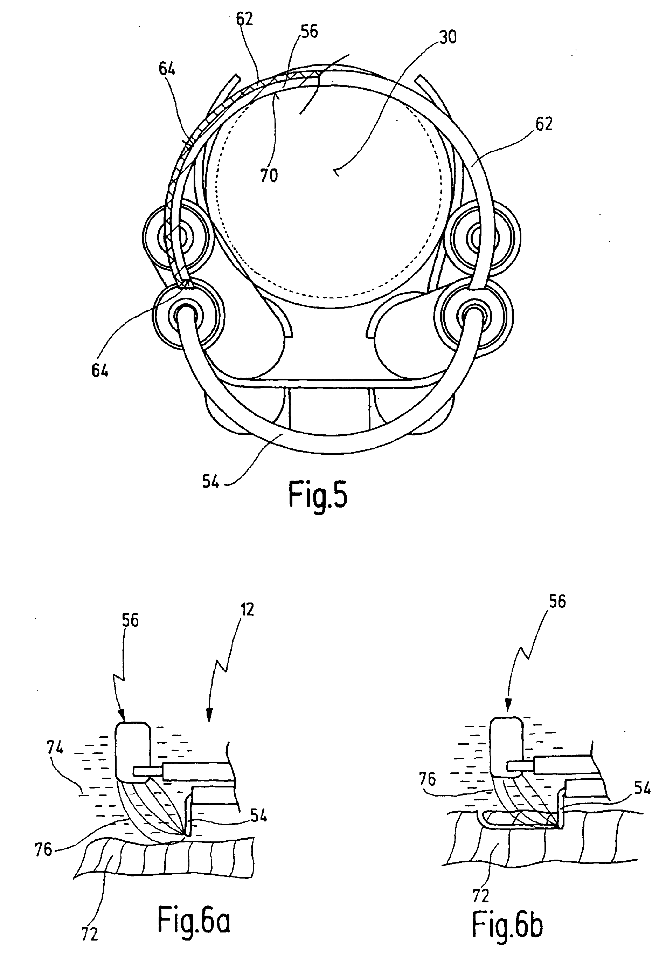

Mit Bezug auf Figuren 2 bis 5 werden nun weitere Einzelheiten der Elektrodenanordnung des Instruments 12 beschrieben. Figuren 2 und 3 zeigen den Elektrodenträger 24 in Alleinstellung, während Fig. 4 den distalen Bereich des Schafts 20 in weiter vergrößerter Darstellung zeigt. Fig. 5 zeigt eine Vorderansicht des distalen Endes des Instruments 12.

Der Elektrodenträger 24 ist aus zwei Stangen 46 und 48 gebildet, die parallel zueinander verlaufen und sich etwa über die Länge des Schafts 20 des Instruments 12 erstrecken. Im Mittelbereich der Stangen 46 und 48 sind diese über Fixierelemente 50 und 52 miteinander verbunden, die im Querschnitt etwa U-förmig ausgebildet sind, und die des weiteren auch die Führung des Elektrodenträgers 24 entlang der Endoskopoptik 22 gewährleisten, wenn der Elektrodenträger 24, wie oben beschrieben, relativ zu der Endoskopoptik bewegt wird.

Distalseitig weist der Elektrodenträger 24 eine Aktivelektrode 54 und eine der Aktivelektrode 54 benachbarte Neutralelektrode 56 auf, die in Fig. 5 durch eine teilweise Aufbrechung der Darstellung zu sehen ist.

Die Neutralelektrode 56 ist vollständig distalseitig der Aktivelektrode 54 angeordnet, d.h. ist in Längsrichtung des Elektrodenträgers 24 gesehen axial von der Aktivelektrode 54 beabstandet und stellt das äußerste distale Ende des Elektrodenträgers 24 dar.

Die Aktivelektrode 54 ist als Drahtschlinge ausgebildet (vgl. Fig. 5), die sich im Wesentlichen senkrecht zur Längsrichtung des Elektrodenträgers 24 erstreckt. Die Neutralelektrode 56 ist als Bandschlinge ausgebildet, wie sich aus einem Vergleich der Figuren 2 bis 4 und 5 ergibt, die sich ebenfalls im Wesentlichen senkrecht zur Längsrichtung des Elektrodenträgers 24 erstreckt, deren Krümmung bzw. konkave Seite jedoch von der Aktivelektrode 54 weg gerichtet ist. Die Neutralelektrode 56 und die Aktivelektrode 54 sind, wie ans den Figuren hervorgeht, in Richtung quer zur Längsrichtung des Elektrodenträgers 24 voneinander beabstandet. Die Neutralelektrode 56 und die Aktivelektrode 54 weisen weder in Längsrichtung des Elektrodenträgers 24 noch in Richtung quer zur Längsrichtung des Elektrodenträgers 24 einen räumlichen Überlappungsbereich auf.

In Fig. 4 ist mit d ein kürzester Abstand zwischen der Aktivelektrode 54 und der Neutralelektrode 56 eingezeichnet, der vorzugsweise im Bereich von 0,5 bis 5 mm, vorzugsweise bei 2,5 mm liegt. Der Versatz zwischen der Neutralelektrode 56 und der Aktivelektrode 54 in Richtung quer zur Längsrichtung des Elektrodenträgers 24 beträgt vorzugsweise im Bereich zwischen etwa 0,5 und etwa 3 mm. Die axiale Erstreckung der Neutralelektrode 56, d.h. die Erstreckung der Neutralelektrode 56 in Längsrichtung des Elektrodenträgers 24 liegt vorzugsweise im Bereich zwischen etwa 1 und etwa 3 mm.

Die Aktivelektrode 54 ist über eine der Stangen, bspw. über die Stange 48, mit einem am proximalen Ende der Stange 48 angeordneten Steckkontakt 58 leitend verbunden, während dann die Neutralelektrode 56 mit dem entsprechenden Steckkontakt 60 am proximalen Ende der Stange 46 leitend verbunden ist. Die Stangen 46 und 48 sind nach außen hin elektrisch isoliert. Die Aktivelektrode 54 kann somit mit dem aktiven Pol des Hochfrequenzgenerators 14 verbunden werden, während die Neutralelektrode 56 mit dem inaktiven Pol des Hochfrequenzgenerators 14 verbunden wird.

Während die Aktivelektrode 54 allseitig frei gelegt ist, d.h. allseitig frei von Isolierungen ist, ist an der Neutralelektrode 56 ein Element 62 vorhanden, das eine elektrisch leitende Berührung einer Außenseite 64 (vgl. Fig. 5) der Neutralelektrode 56 durch Gewebe verhindert. Beispielsweise ist das Element 62 unmittelbar an der Neutralelektrode 56 befestigt, so dass es sich bei einer Positionsveränderung der Neutralelektrode 56, bspw. wenn die Handhabe 34 betätigt wird, mit der Neutralelektrode 56 mit bewegt. Alternativ kann das Element 62 jedoch auch mit dem Elektrodenträger 24 verbunden sein und die Neutralelektrode 56 einfach überdecken.

Das Element 62 ist elektrisch isolierend, und besteht bspw. aus einem keramischen Werkstoff oder aus einem Kunststoff. Besonders bevorzugt ist es, wenn das Element 62 in Form einer Beschichtung der Neutralelektrode 56 mit Polyetheretherketon (PEEK) aufgebracht ist. Das Element 62 überdeckt die gesamte Außenseite 64 der Neutralelektrode 56 vollflächig, und zwar auch an einer distalen Stirnseite 66 und einer proximalen Stirnseite 68 der Neutralelektrode 56. Das Element 62 verhindert, dass die Neutralelektrode 56 außenseitig mit unbeteiligtem Gewebe in Berührung kommt, so dass eine Beschädigung von unbeteiligtem Gewebe durch die Neutralelektrode 56 verhindert wird.

Demgegenüber ist die Neutralelektrode 56 auf ihrer der Aktivelektrode 54 zugewandten Innenseite 70 (vgl. Fig. 5) nicht isoliert, so dass sich zwischen der Aktivelektrode 54 und der Innenseite 70 der Neutralelektrode 56 ein Stromfluss ausbilden kann, wenn sich zwischen der Innenseite 70 der Neutralelektrode 56 und der Aktivelektrode 54 Gewebe und/oder eine elektrisch leitende Flüssigkeit, bspw. eine Spülflüssigkeit, für die eine isotonische Kochsalzlösung verwendet wird, befindet.

Die Isolierung der Aktivelektrode 54 kann, wie in Fig. 4 mit gestrichelten Linien dargestellt ist, sich noch weiter zum äußeren Ende der Aktivelektrode 54 hin erstrecken, so dass sich an dem äußeren Ende der Aktivelektrode 54 eine höhere Stromdichte ergibt, wodurch die Schneidwirkung der Aktivelektrode 54 an ihrem äußeren Ende noch verbessert werden kann.

Mit Bezug auf Fig. 6 und Fig. 1 wird nachfolgend die Funktionsweise des Instruments 12 und des Hochfrequenzgenerators 14 näher beschrieben.

Der Hochfrequenzgenerator 14 ist in der Lage, zumindest zeitweise eine Ausgangsleistung von zumindest 200 Watt abzugeben.

In Fig. 6a) ist mit dem Bezugszeichen 72 Gewebe dargestellt, in das mit dem Instrument 12 ein Gewebeschnitt eingebracht werden soll. Die Aktivelektrode 54 und die Neutralelektrode 56 sind dabei von einer elektrisch leitenden Flüssigkeit umgeben, da bei solchen Vorgängen mit einer isotonischen Kochsalzlösung gespült wird.

Wird die Elektrode 54, wie in Fig. 6a) dargestellt, auf das Gewebe 72 aufgesetzt und der Hochfrequenzgenerator 14 eingeschaltet, bildet sich zunächst auf Grund der höheren Impedanz des Gewebes 72 im Vergleich zu der Flüssigkeit 74 ein Stromfluss zwischen der Aktivelektrode 54 und der Neutralelektrode 56 aus, wie mit Stromlinien 76 in Fig. 6a) angedeutet ist. Der Stromfluss geht in diesem Zustand nicht oder allenfalls geringfügig durch das Gewebe 72 hindurch. Um einen für ein elektrisches Schneiden ausreichenden Stromfluss durch das Gewebe 72 während dieses Anschneidens zu gewährleisten, wird nun temporär die Ausgangsleistung des Hochfrequenzgenerators auf zumindest 200 Watt erhöht, und zwar während einer Dauer von etwa 0,2 s (bei einer Frequenz von 150-350 kHz). Nunmehr ist es der Aktivelektrode 54 möglich, in das Gewebe 72 einzudringen und darin einen Schnitt auszuführen, wie in Fig. 6b) dargestellt ist.

Durch Betätigen der Handhabe 34 wird nun die Aktivelektrode 54 und mit ihr die mit ihr mechanisch über den Elektrodenträger 24 verbundene Neutralelektrode 56 nach proximal gezogen, um das Gewebe 72 zu schneiden. Bei diesem Weiterschneidvorgang wird die Leistung des Hochfrequenzgenerators 14 wieder vermindert, und zwar unter 200 Watt, um eine Carbonisierung des Gewebes 72 zu vermeiden. Eine Erhöhung der Ausgangsleistung des Hochfrequenzgenerators 14 ist nun auch nicht mehr erforderlich, da sich der Stromfluss zwischen der Aktivelektrode 54 und der Neutralelektrode 56 auch ausreichend durch das Gewebe 72 hindurch erstreckt. Falls jedoch auch beim Weiterschneiden der Stromfluss zwischen der Aktivelektrode 54 und der Neutralelektrode 56 abbricht, kann wieder temporär die Ausgangsleistung des Hochfrequenzgenerators 14 erhöht werden, bis wieder ein Stromfluss vorhanden ist.

## Patentansprüche

1. Bipolares medizinisches Instrument zum Schneiden von Gewebe (72) unter der Wirkung von Hochfrequenzstrom, mit einem sich längs erstreckenden Elektrodenträger (24), einer an dem Elektrodenträger (24) distalseitig angeordneten Aktivelektrode (54) und einer dieser benachbarten Neutralelektrode (56), wobei die Aktivelektrode (54) als Drahtschlinge ausgebildet ist und wobei die Neutralelektrode (56) vollständig distalseitig der Aktivelektrode (54) angeordnet ist, **dadurch gekennzeichnet, dass** die Neutralelektrode (56) und die Aktivelektrode (54) in Richtung quer zur Längsrichtung des Elektrodenträgers (24) ohne einen räumlichen Überlappungsbereich voneinander beabstandet sind.

2. Instrument nach Anspruch 1, **dadurch gekennzeichnet, dass** die Neutralelektrode (56) als Bandschlinge ausgebildet ist, vorzugsweise mit von der Aktivelektrode (54) weg gerichteter Krümmung.

3. Instrument nach Anspruch 1 oder 2, **dadurch gekennzeichnet, dass** am Elektrodenträger (24) und/oder an der Neutralelektrode (56) zumindest ein Element (62) vorhanden ist, das eine elektrisch leitende Berührung einer der Aktivelektrode (54) abgewandten Außenseite (64) der Neutralelektrode (56) durch Gewebe verhindert, und das sich bei einer Positionsveränderung der Neutralelektrode (56) mit dieser mitbewegt.

4. Instrument nach Anspruch 3, **dadurch gekennzeichnet, dass** das zumindest eine Element (62) selbst elektrisch isolierend ist.

5. Instrument nach Anspruch 3 oder 4, **dadurch gekennzeichnet, dass** das zumindest eine Element (62) aus einem keramischen Werkstoff oder aus einem Kunststoff ist.

6. Instrument nach einem der Ansprüche 3 bis 5, **dadurch gekennzeichnet, dass** das Element (62) durch eine Beschichtung der Neutralelektrode (56) gebildet ist.

7. Instrument nach einem der Ansprüche 3 bis 6, **dadurch gekennzeichnet, dass** das zumindest eine Element (62) unmittelbar auf der der Aktivelektrode (54) abgewandten Außenseite (66) der Neutralelektrode (56) befestigt ist.

8. Instrument nach einem der Ansprüche 3 bis 7, **dadurch gekennzeichnet, dass** das zumindest eine Element (62) die Außenseite der Neutralelektrode (56) vollflächig bedeckt.

9. Instrument nach einem der Ansprüche 3 bis 8, **dadurch gekennzeichnet, dass** das zumindest eine Element (62) eine elektrisch leitende Berührung der distalseitigen und oder proximalseitigen Stirnseite (66, 68) der Neutralelektrode (56) mit Gewebe verhindert.

10. Instrument nach Anspruch 9 **dadurch gekennzeichnet, dass** das zumindest eine Element (62) die distalseitige und/oder proximalseitige Stirnseite (66, 68) der Neutralelektrode (56) vollflächig bedeckt.

11. Elektrochirurgisches System, mit einem Hochfrequenzgenerator (14), **gekennzeichnet durch** ein medizinisches Instrument (12) nach einem der Ansprüche 1 bis 10, das an den Hochfrequenzgenerator (14) anschließbar ist.

12. System nach Anspruch 11, **dadurch gekennzeichnet, dass** der Hochfrequenzgenerator (14) in der Lage ist, eine Ausgangsleistung von zumindest 200 W abzugeben.

13. System nach Anspruch 11 oder 12, **dadurch gekennzeichnet, dass** der Hochfrequenzgenerator (14) eine Leistungsregelung aufweist, derart, dass die von dem Hochfrequenzgenerator (14) abgegebene Leistung vor und während des Eindringens der Aktivelektrode (54) in Gewebe (72) (Anschneiden) höher ist als beim Weiterschneiden, wenn die Aktivelektrode (54) in das Gewebe eingedrungen ist.

## Claims

1. A bipolar medical instrument for cutting tissue (72) under the action of high-frequency current, comprising an elongate electrode carrier (24), an active electrode (54) arranged at the distal end of the electrode carrier (24), and a neutral electrode (56) adjacent to the active electrode (54), wherein the active electrode (54) is configured as a wire loop and wherein said neutral electrode (56) being arranged entirely to the distal side of said active electrode (54), **characterized in that** the neutral electrode (56) and the active electrode (54) are spaced apart from one another in the direction transverse to the longitudinal direction of the electrode carrier (24) without spatial overlap.

2. The instrument of claim 1, **characterized in that** the neutral electrode (56) is configured as a tape loop, preferably with a curvature oriented away from the active electrode (54).

3. The instrument of claim 1 or 2, **characterized in that**, on the electrode carrier (24) and/or on the neutral electrode (56), at least one element (62) is present which prevents electrically conductive contact between tissue and an outer face (64) of the neutral electrode (56) directed away from the active electrode (54), and which, when the neutral electrode (56) changes position, moves along with said neutral electrode.

4. The instrument of claim 3, **characterized in that** the at least one element (62) is itself electrically insulating.

5. The instrument of claim 3 or 4, **characterized in that** the at least one element (62) is made from a ceramic material or from a plastic.

6. The instrument of anyone of claims 3 through 5, **characterized in that** the element (62) is formed by a coating of the neutral electrode (56).

7. The instrument of anyone of claims 3 through 6, **characterized in that** the at least one element (62) is secured directly on the outer face (66) of the neutral electrode (56) directed away from the active electrode (54).

8. The instrument of anyone of claims 3 through 7, **characterized in that** the at least one element (62) completely covers the outer face of the neutral electrode (56).

9. The instrument of anyone of claims 3 through 8, **characterized in that** the at least one element (62) prevents electrically conductive contact between the distal and/or proximal end face (66, 68) of the neutral electrode (56) and tissue.

10. The instrument of claim 9, **characterized in that** the at least one element (62) covers the complete area of the distal and/or proximal end face (66, 68) of the neutral electrode (56).

11. An electrosurgical system, comprising a high-frequency generator (14), **characterized by** a medical instrument (12) as claimed in anyone of claims 1 through 10, which instrument can be connected to the high-frequency generator (14).

12. The system of claim 11, **characterized in that** the high-frequency generator (14) is able to output power of at least 200 W.

13. The system of claim 11 or 12, **characterized in that** the high-frequency generator (14) has an output regulator which is such that the power output by the high-frequency generator (14) before and during penetration of the active electrode (54) into tissue (72) (initial cutting) is greater than during the continued cutting when the active electrode (54) has penetrated into the tissue.

## Revendications

1. Instrument médical bipolaire destiné à couper un tissu (72) sous l'effet d'un courant haute fréquence, comprenant un support d'électrode (24) s'étendant longitudinalement, une électrode active (54) disposée du côté distal sur le support d'électrode (24), et une électrode neutre (56) adjacente à celle-ci, l'électrode active (54) étant réalisée comme une boucle en fil métallique, et l'électrode neutre (56) étant disposée complètement du côté distal de l'électrode active (54), **caractérisé en ce que** l'électrode neutre (56) et l'électrode active (54) sont espacées l'une de l'autre dans la direction transversale à la direction longitudinale du support d'électrode (24) sans zone de recouvrement spatial.

2. Instrument selon la revendication 1, **caractérisé en ce que** l'électrode neutre (56) est réalisée comme une boucle en bande, de préférence avec une courbure détournée de l'électrode active (54).

3. Instrument selon la revendication 1 ou 2, **caractérisé en ce que** sur le support d'électrode (24) et/ou sur l'électrode neutre (56), il existe au moins un élément (62) qui empêche un contact de conduction électrique d'un côté extérieur (64) de l'électrode neutre (56), détourné de l'électrode active (54), par un tissu, et qui, en cas de modification de position de l'électrode neutre (56), se déplace avec celle-ci.

4. Instrument selon la revendication 3, **caractérisé en ce que** ledit au moins un élément (62) est lui-même électriquement isolant.

5. Instrument selon la revendication 3 ou 4, **caractérisé en ce que** ledit au moins un élément (62) est réalisé à partir d'un matériau céramique ou d'une matière plastique.

6. Instrument selon l'une quelconque des revendications 3 à 5, **caractérisé en ce que** l'élément (62) est réalisé par un revêtement de l'électrode neutre (56).

7. Instrument selon l'une quelconque des revendications 3 à 6, **caractérisé en ce que** ledit au moins un élément (62) est fixé directement sur le côté extérieur (66) de l'électrode neutre (56), détourné de l'électrode active (54).

8. Instrument selon l'une quelconque des revendications 3 à 7, **caractérisé en ce que** ledit au moins un élément (62) recouvre le côté extérieur de l'électrode neutre (56) sur toute la surface.

9. Instrument selon l'une quelconque des revendications 3 à 8, **caractérisé en ce que** ledit au moins un élément (62) empêche un contact de conduction électrique entre le côté frontal (66, 68) de l'électrode neutre (56), du côté distal et/ou du côté proximal, et un tissu.

10. Instrument selon la revendication 9, **caractérisé en ce que** ledit au moins un élément (62) recouvre le côté frontal (66, 68) de l'électrode neutre (56), du côté distal et/ou du côté proximal, sur toute la surface.

11. Système électrochirurgical, comprenant un oscillateur haute fréquence (14), **caractérisé par** un instrument médical (12) selon l'une quelconque des revendications 1 à 10 qui peut être connecté à l'oscillateur haute fréquence (14).

12. Système selon la revendication 11, **caractérisé en ce que** l'oscillateur haute fréquence (14) est capable de délivrer une puissance de sortie d'au moins 200 W.

13. Système selon la revendication 11 ou 12, **caractérisé en ce que** l'oscillateur haute fréquence (14) présente une régulation de puissance de telle sorte que la puissance délivrée par l'oscillateur haute fréquence (14) est supérieure avant et pendant la pénétration de l'électrode active (54) dans un tissu (72) (entame) que pendant la suite de la découpe lorsque l'électrode active (54) a pénétré dans le tissu.
